(19) Europäisches Patentamt
European Patent Office
Office européen des brevets

(11) **EP 3 437 501 A1**

(12) **EUROPEAN PATENT APPLICATION**
published in accordance with Art. 153(4) EPC

(43) Date of publication:
**06.02.2019 Bulletin 2019/06**

(21) Application number: **17774809.2**

(22) Date of filing: **24.03.2017**

(51) Int Cl.:
**A41D 13/05** (2006.01)　　　**A41D 13/08** (2006.01)
**A61H 3/00** (2006.01)

(86) International application number:
**PCT/JP2017/012154**

(87) International publication number:
**WO 2017/170272 (05.10.2017 Gazette 2017/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **30.03.2016　JP 2016069759**

(71) Applicant: **Toray Industries, Inc.**
**Tokyo 103-8666 (JP)**

(72) Inventors:
• **CHIN, Takaaki**
**Kobe-shi**
**Hyogo 651-2134 (JP)**

• **HONDA, Yuichiro**
**Kobe-shi**
**Hyogo 651-2134 (JP)**
• **NAKAMURA, Go**
**Kobe-shi**
**Hyogo 651-2134 (JP)**
• **KASABO, Miki**
**Chuo-ku, Tokyo 103-8666 (JP)**
• **OKAZAKI, Osamu**
**Osaka-shi**
**Osaka 530-8222 (JP)**

(74) Representative: **Kador & Partner PartG mbB**
**Corneliusstraße 15**
**80469 München (DE)**

(54) **GARMENT**

(57) The present invention provides a garment suitable for individuals with reduced physical ability and those at risk of suffering from reduction in physical ability, the garment being capable of supporting joint movement produced by the wearer's own muscles and effectively preventing loss of muscle strength to maintain and enhance the wearer's daily physical activity. The garment (20a) comprises a body covering (21a) and support belts (2a, 2b) arranged on the body covering (21a), the body covering (21a) comprising portions for covering joints of a wearer, wherein the support belts (2a, 2b) are arranged on the body covering (21a) to run along a surface of the body of the wearer so that each of the joint-covering portions of the body covering (21a) is positioned between one end of either of the support belts (2a, 2b) and the opposite end thereof, wherein the support belts (2a, 2b) are attached to the body covering (21a) so that the support belts (2a, 2b) and the body covering (21a) are separately and individually stretchable, and wherein the support belts (2a, 2b) each have a tension (T1) in a direction (S) connecting said one end (3a) and said opposite end (3b) of either of the support belts (2a, 2b), and portions of the body covering (21a) that lie below the support belts (2a, 2b) each have a tension (T2) in a direction parallel to the direction (S), and the tension (T1) is larger than the tension (T2) when the garment (20a) is worn.

Fig. 1

**Description**

TECHNICAL FIELD

The present invention relates to a garment suitable for individuals with reduced physical ability and those at risk of suffering from reduction in physical ability, the garment being capable of supporting joint movement produced by the wearer's own muscles and effectively preventing loss of muscle strength to maintain and enhance the wearer's daily physical activity.

BACKGROUND ART

The world population is rapidly aging. Aging society poses large social challenges in terms of how to encourage aging adults to work longer depending on their physical ability and to live independent and stay active while reducing soaring medical costs and social security costs.

Enhancement of muscular strength is an important factor for maintaining and enhancing the physical ability to manage daily life. In the elderly, reduction of muscular strength imposes a significant burden on the joints and also causes difficulties in daily activities, such as changing or maintaining body position. Increasing body flexibility will help improve the range of motion of joints and joint functions and release the tension of muscles, which will lead to the prevention of injuries. In particular, supporting joint movement so as to reproduce the natural motion of the body, such as that in walking, is an important approach to the maintenance of the physical ability of healthy individuals and to rehabilitation for functional recovery of those with reduced physical ability.

Various types of sportswear and sports underwear as well as various types of garments with supporting functions have been proposed for those with normal physical ability or a normal activity level, or those with relatively high physical ability or a relatively high activity level. Examples of such garments known in the art include a garment that applies varying pressure to different parts of a lower limb (see Patent Literature 1), a garment that applies increased compression force to a particular body part (see Patent Literature 2), a garment made of a combination of materials that are stretchable in different directions (see Patent Literature 3), and a garment having reinforcements disposed along the muscles or ligaments of the wearer (see Patent Literature 4). These types of garments are collectively called compression garments for their ability to apply pressure.

These compression garments, however, are designed to apply pressure to specific muscles, and the purpose of applying pressure is to protect or enhance the muscles, rather than to directly support joint movement. Another problem is that the compression force applied by the compression garments is generally excessively strong, which makes them difficult to put on for individuals with reduced physical ability or those at risk of suffering from reduction in physical ability, due to lack of grip strength. For the same reason, the compression garments are difficult to wear for a long period of time, especially for the elderly who tend to have an increased waist size as the age advances. Among individuals with reduced physical ability and those at risk of suffering from reduction in physical ability, there are many individuals with very low muscular strength, including, for example, ordinary individuals of middle and advanced age who do not do regular exercise. For these individuals, doing regular exercise wearing any of the above compression garments in an attempt to enhance the muscular strength requires considerable willpower and endurance, and thus such exercise is often daunting.

For such individuals with reduced physical ability and low muscular strength, a powered assistive orthosis that supports walking with a little assistance for the swing of the legs around the hip joint as described in Patent Literature 5 and 6 has been proposed by various automakers, material suppliers and universities.

CITATION LIST

PATENT LITERATURE

Patent Literature 1: JP H10-130915 A
Patent Literature 2: JP H10-280209 A
Patent Literature 3: JP 2006-219778 A
Patent Literature 4: JP 2013-227717 A
Patent Literature 5: JP 2009-095645 A
Patent Literature 6: JP 2014-018536 A

SUMMARY OF INVENTION

TECHNICAL PROBLEM

The assistive orthosis as described in Patent Literature 5 and 6, however, is electrically powered and thus requires a battery. The capacity of current lithium ion batteries is not large enough. Consequently a battery adds a huge weight to the assistive orthosis and imposes limitations on, for example, the number of hours during which the wearer can use the assistive orthosis. The assistive orthosis is also troublesome to put on because it requires, every time the wearer puts it on, electronic setting for the supporting force and mechanical adjustment as appropriate for the wearer's physical ability by a specialist.

The present invention was made to solve the above problems. An object of the present invention is to provide a garment that requires no electric energy source, is produced at a low cost, is comfortable to wear and highly safe, and effectively supports joint movement in the body so as to reproduce the natural motion of a human body.

SOLUTION TO PROBLEM

The inventors conducted extensive research to solve the above problems, and found that the above problems are solved by the following means.

(1) A garment comprising a body covering and a support belt arranged on the body covering, the body covering comprising a portion for covering a joint of a wearer,
wherein the support belt is arranged on the body covering to run along a surface of the body of the wearer so that the joint-covering portion of the body covering is positioned between one end of the support belt and the opposite end thereof,
wherein the support belt is attached to the body covering so that the support belt and the body covering are separately and individually stretchable, and
wherein the support belt has a tension T1 in a direction S connecting said one end and said opposite end of the support belt, and a portion of the body covering that lies below the support belt has a tension T2 in a direction parallel to the direction S, and the tension T1 is larger than the tension T2 when the garment is worn.
(2) The garment according to the above (1), which has a ratio of the tension T1 to the tension T2 (T1/T2) ranging from 3 to 100.
(3) The garment according to the above (1) or (2), which further has a means for adjusting the tension T1.
(4) The garment according to any one of the above (1) to (3), which comprises two support belts A1, A2,
wherein the body covering comprises a portion A for covering the waist of the wearer, a portion B for covering the right knee of the wearer and a portion C for covering the left knee of the wearer,
wherein one end of the support belt A1 is detachably attached to the portion A, and the opposite end of the support belt A1 is detachably attached to the portion B, and
wherein one end of the support belt A2 is detachably attached to the portion A, and the opposite end of the support belt A2 is detachably attached to the portion C.
(5) The garment according to any one of the above (1) to (4), which further comprises an adjusting belt,
wherein the body covering comprises a portion A for covering the waist of the wearer and a portion for covering a region around the right and left iliac crests of the wearer when the garment is worn,
wherein the adjusting belt is overlaid on the iliac crest-covering portion of the body covering, and
wherein the garment has a hip fastening member that detachably attaches the adjusting belt to at least part of the portion A.
(6) The garment according to any one of the above (1) to (3), wherein the body covering is a tubular body comprising a portion D for covering an upper arm of the wearer, a portion E for covering an elbow of the wearer, and a portion F for covering a wrist of the wearer, and
wherein one end of the support belt is detachably attached to the portion D, and the opposite end of the support belt is detachably attached to the portion F.

ADVANTAGEOUS EFFECTS OF INVENTION

The present invention provides a garment suitable for individuals with reduced physical ability and those at risk of suffering from reduction in physical ability, the garment being capable of supporting physical activity of the wearer to motivate the wearer to stay physically active, thereby effectively preventing loss of muscle strength. The garment requires no electric energy source, is produced at a low cost, is comfortable to wear and highly safe, and effectively supports the body motion so as to reproduce the natural motion of a human body.

BRIEF DESCRIPTION OF DRAWINGS

Fig. 1 is a schematic front view of a garment according to an embodiment of the present invention when it is worn.
Fig. 2 is a schematic front view of a garment according to another embodiment of the present invention when it is worn.
Fig. 3 is a schematic front view of a garment according to another embodiment of the present invention when it is worn.
Fig. 4 is a schematic front view of a garment according to another embodiment of the present invention when it is worn.
Fig. 5 is a schematic rear view of a garment according to another embodiment of the present invention when it is worn.
Fig. 6 (a) is a schematic left side view of a body covering according to an embodiment of the present invention, used in the garment of the present invention as shown in Figs. 3 and 4. Fig. 6 (b) is a schematic front view of the body covering according to the embodiment of the present invention, used in the garment of the present invention as shown in Figs. 3 and 4. Fig. 6 (c) is a schematic right side view of the body covering according to the embodiment of the present invention, used in the garment of the present invention as shown in Figs. 3 and 4.
Fig. 7 (a) and (b) is a schematic front view of support belts according to an embodiment of the present invention, used in the garment of the present invention as shown in Figs. 1 to 5.
Fig. 8 (a) is a schematic left side view of a body covering according to an embodiment of the present invention, used in the garment of the present invention as shown in Figs. 1 and 2. Fig. 8 (b) is a schematic front view of the body covering according to the embodiment of the present invention, used in the garment of the present invention as shown in Figs. 1 and 2. Fig. 8 (c) is a schematic right side view of the body covering according to the embodiment of the present invention, used in the garment of the present invention as shown in Figs. 1 and 2.
Fig. 9 (a) is a schematic left side view of a body covering according to an embodiment of the present invention, used in the garment of the present invention as shown in Fig. 5. Fig. 9 (b) is a schematic front view of the body covering according to the embodiment of the present invention, used in the garment of the present invention as shown in Fig. 5. Fig. 9 (c) is a schematic right side view of the body covering according to the embodiment of the present invention, used in the garment of the present invention as shown in Fig. 5.
Fig. 10 is a schematic right side view of a garment according to another embodiment of the present invention when it is worn.
Fig. 11 is a schematic right side view of a body covering according to an embodiment of the present invention, used in the garment of the present invention as shown in Fig. 10.
Fig. 12 is a schematic developed view showing the pieces of the body covering used in the embodiment of the garment of the present invention as shown in Fig. 6 (a), (b) and (c).
Fig. 13 is a schematic developed view showing the pieces of the body covering used in the embodiment of the garment of the present invention as shown in Fig. 8 (a), (b) and (c).
Fig. 14 (a) is a schematic front view of a garment according to another embodiment of the present invention when it is worn. Fig. 14 (b) is a schematic right side view of the garment according to the embodiment of the present invention when it is worn. Fig. 14 (c) is a schematic rear view of the garment according to the embodiment of the present invention when it is worn.
Fig. 15 is a schematic front view of an adjusting belt according to an embodiment of the present invention, used in the garment of the present invention as shown in Fig. 14 (a), (b) and (c).

DESCRIPTION OF EMBODIMENTS

The garment of the present invention is a garment comprising a body covering and a support belt arranged on the body covering, the body covering comprising a portion for covering a joint of a wearer, wherein the support belt is arranged on the body covering to run along a surface of the body of the wearer so that the joint-covering portion of the body covering is positioned between one end of the support belt and the opposite end thereof. The support belt is attached to the body covering so that the support belt and the body covering are separately and individually stretchable. The support belt has a tension T1 in a direction S connecting said one end and said opposite end of the support belt, and a portion of the body covering that lies below the support belt has a tension T2 in a direction parallel to the direction S, and the tension T1 is larger than the tension T2 when the garment is worn.
Said one end and said opposite end of the support belt are located on the distal and proximal sides of a joint of a wearer in need, respectively. The support belt is attached to the body covering while moderate tension is applied to the support belt so that the tension T1 is larger than the tension T2. Due to this arrangement, when the joint is moved, the support belt exhibits resilient force to return to its original shape, thereby guiding the joint motion toward the desired direction at the initial stage of the movement. In the garment of the present invention, the body covering and the support belt that are located over a joint of the wearer in need are separately and independently stretchable. The movement of the support belt is not restrained except for the location where the support belt is attached to the body covering. The support belt does not inhibit the elongation of the body covering in the area where the support belt overlaps the joint-covering portion of the body covering. Therefore the garment of the present invention gives less tight feel as compared with a conventional compression garment, and due to this, the wearer can wear the garment of the present invention continuously for a long period of time. Since the support belt and the body covering are separately and independently stretchable, said one end and said opposite end of the support belt directly pull each other, and the pulling force is prevented from dispersing and is easily and directly transferred to the joint of the wearer. In order to efficiently utilize the support belt's resilient force, the coefficient of static friction between the body covering and the non-attached portion of the support belt is desirably low.

The garment of the present invention supports a joint, in particular, an upper limb joint, such as a shoulder joint, an elbow joint, a wrist joint, a hand joint, a finger joint and a forearm joint; and a lower limb joint, such as a hip joint, a knee joint, an ankle joint, a toe joint, a cervical vertebral joint, and a thoracolumbar joint. In other words, the joint to be supported by the garment is not particularly limited, and may be any place where two bones are joined together. The support belt may be overlaid on a plurality of joints. Each of the above listed joints has a defined range of motion that is expressed in the direction and degrees of motion, such as flexion, extension, adduction, abduction, internal rotation, external rotation, pronation and supination. The support belt is desirably arranged to achieve the desired movement of a joint in the desired body part as appropriate for the conditions of the wearer.

A plurality of the support belts may be provided to the garment of the present invention. The support belts may be arranged on different positions or stacked on top of each other.

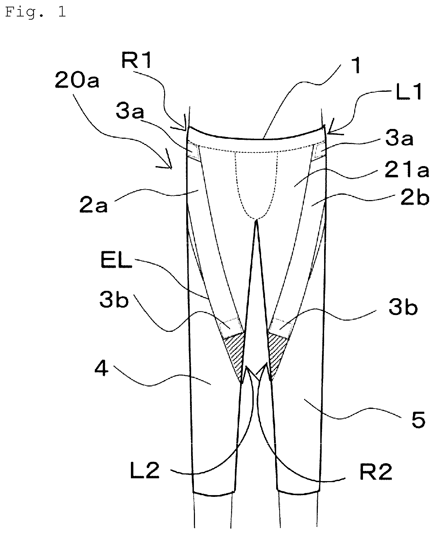

Fig. 1 shows a schematic front view of the garment according to an embodiment of the present invention when it is worn. The garment 20a comprises a body covering 21a and two support belts 2a, 2b. The body covering 21a comprises a portion (A) 1 for covering the waist of the wearer when the garment is worn, a portion (B) 4 for covering the right knee of the wearer when the garment is worn, a portion (C) 5 for covering the left knee of the wearer when the garment is worn, and four fastening members to be paired with the corresponding fastening members on the support belts. The support belts 2a, 2b each have two fastening members to be paired with the corresponding fastening members on the body covering. That is, the fastening members on the support belts 2a, 2b are paired with and fastened to the corresponding fastening members on the body covering 21a, and thereby the support belts 2a, 2b are detachably attached to the body covering 21a.

The four fastening members on the body covering 21a to be paired with the corresponding fastening members on the support belts are each disposed on the following different locations on the body covering 21a: the right side R1 and the left side L1 of the portion A for covering the waist, the left side L2 of the portion B for covering the right knee, and the right side R2 of the portion C for covering the left knee. The two fastening members on each of the support belts 2a, 2b to be paired with the corresponding fastening members on the body covering are disposed on one end 3a of each of the support belts and the opposite end 3b thereof, respectively.

The support belt 2a is detachably attached to the body covering by fastening the fastening member disposed on said one end 3a of the support belt 2a to the fastening member disposed on the right side R1 of the portion A for covering the waist and by fastening the fastening member disposed on said opposite end 3b of the support belt 2a to the fastening member disposed on the left side L2 of the portion B for covering the right knee. The support belt 2b is detachably attached to the body covering by fastening the fastening member disposed on said one end 3a of the support belt 2b to the fastening member disposed on the left side L1 of the portion A for covering the waist and by fastening the fastening member disposed on said opposite end 3b of the support belt 2b to the fastening member disposed on the right side R2 of the portion C for covering the left knee.

The two support belts 2a, 2b are arranged so that the hip joint of the wearer is located between the two ends 3a, 3b of each of the support belts and the support belts run along the anterior surface of the thighs of the wearer. The support belts 2a, 2b arranged on the surface of the body covering 21a each have the tension T1 in the direction S connecting said one end 3a and said opposite end 3b of either of the support belts 2a, 2b, and the portions of the body covering 21a that lie below the support belts each have the tension T2 in the direction parallel to the direction S, and the tension T1 is larger than the tension T2, as described above. Due to this configuration, the garment of the present invention appropriately supports the flexion of the hip joint of the wearer, thereby providing support for walking of the wearer.

The garment of the present invention having the structure as exemplified above requires no electric energy source and is easy to produce at a low cost. The garment of the present invention is a piece of clothing and is therefore safer and more lightweight than an electrically powered assistive orthosis and is highly safe.

The ratio of the tension T1 to the tension T2 (T1/T2) in the garment of the present invention preferably ranges from 3 to 100. The support belt having a T1/T2 ratio of 3 or more exhibits sufficient resilient force to return to its original shape, thereby appropriately guiding the joint motion toward the desired direction. The support belt having a T1/T2 ratio of 100 or less exhibits moderate pulling force between the two opposite ends, and as a result, the garment is more comfortable to wear, and is capable of reducing the wearer's fatigue from maintaining the posture and body position to wait until joint motion is guided. More preferably, the T1/T2 ratio is 7 or more at the minimum, and is 80 or less at the maximum.

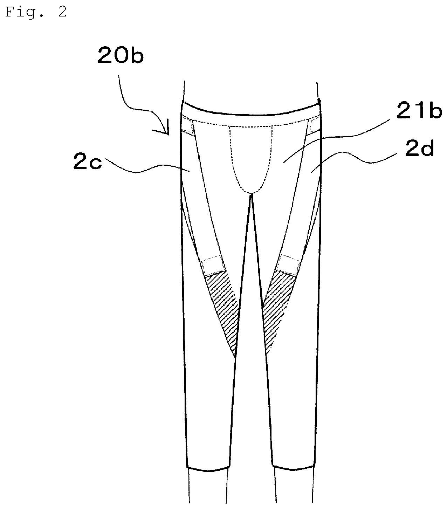

Fig. 2 shows a schematic front view of the garment according to another embodiment of the present invention when it is worn. The garment 20b is made of the same members as those constituting the garment 20a of Fig. 1, but the garment 20b differs from the garment 20a of Fig. 1 in that the support belts 2c, 2d are attached to the body covering 21b so that the tension T1 of the support belts 2c, 2d is smaller than that of the support belts 2a, 2b of the garment 20a of Fig. 1. Typically, for example, when one support belt is pulled by any given force in the longitudinal direction and an identical support belt is pulled by a smaller force than that in the longitudinal direction, the tension of the latter support belt will be smaller. Such a difference in the tension between the garment 20a of Fig. 1 and the garment 20b of Fig. 2 can be realized by providing a means for adjusting the tension T1 to the garment of the present invention.

The garment provided with a means for adjusting the tension T1 of the support belt allows the wearer, for example, to reduce the tension at the early stage of exercise training and to later gradually increase or reduce the tension to a level where the wearer feels most appropriate to them after the wearer gets used to wearing the garment. In this manner, the wearer is able to find the optimal position of the support belt. The conditions and physical ability of the wearer may vary depending on the season and the time of the day. Therefore the adjustment of the tension in this manner is preferable in that the wearer is able to obtain supporting force appropriate to their physical ability at the time of wearing. Another benefit is that the wearer is able to adjust the tension balance between the right and left sides of the body in cases where there are differences in the functional ability between the right and left extremities . This is a great advantage over many of conventional compression garments, which merely have a reinforcement over a muscle or a ligament of the wearer and are incapable of adjusting the tension as appropriate to the physical ability of the wearer after it is put on or other occasions. The advantage of being able to adjust the tension as appropriate to the physical ability of the wearer will help develop the habit of putting the garment on and establish the routine of regular exercise for joints. The degree of support for joints provided by the garment of the present invention is adjustable as appropriate to the physical ability of the wearer in the above described manner, and the garment thereby facilitates smooth joint movement.

The means for adjusting the tension T1 may be, for example, either of the following two means: adjusting the length of the support belt in the absence of longitudinal tension; and adjusting the distance between the positions of attachment of the opposite ends of the support belt to the body covering. For the former means (for adjusting the length of the support belt), a buckle or an adjuster having a common width, made of a material commonly used for clothing auxiliary materials, such as nylon and polyacetal, can be suitably employed. By using a buckle or an adjuster, the length of the non-attached portion of the support belt can be adjusted. A longer length of the non-attached portion of the support belt will result in a smaller tension T1, whereas a shorter length of the non-attached portion of the support belt will result in a higher tension T1. For the latter means (for adjusting the distance between the positions of attachment of the opposite ends of the support belt to the body covering), an auxiliary material capable of fastening a part of the support belt to the body covering, such as a hook and loop fastener and a snap fastener, can be employed. For example, a hook or loop portion of a hook and loop fastener is provided to at least one end of the support belt, and the corresponding hook or loop portion of greater length and width is provided to the corresponding part of the body covering. By adjusting the position of attachment of the hook or loop portion of said one end of the support belt to the corresponding hook or loop portion of the body covering, the distance between the positions of attachment of the opposite ends of the support belt to the body covering can be adjusted, thereby easily adjusting the tension T1.

The term "hook and loop fastener" refers to a joinder means containing two surfaces capable of being joined together when pressed together, and the two surfaces can easily be pulled apart and rejoined over and over again. Most of common hook and loop fasteners consist of a hook surface and a loop surface, but according to the present invention, preferred hook and loop fasteners also include a hook and loop fastener consisting of two identical surfaces that are covered with hooks and loops, a hook and loop fastener capable of creating a strong bond due to its mushroom-shaped hooks or saw-tooth-shaped hooks, and other types of hook and loop fasteners. Some types of hook and loop fasteners are stretchable and thus conformable to accommodate body movement during exercise. Due to this advantage, either of the paired strips of the hook and loop fastener is preferably a stretchable type. The tension A will be larger when the distance between the positions of attachment of the opposite ends of the support belt is larger, i.e., when the support belt is pulled more tightly and attached to the body covering. On the other hand, the tension A will be smaller when the distance between the positions of attachment of the opposite ends of the support belt is smaller, i.e., when the support belt is pulled less tightly and attached to the body covering.

The timing of adjusting the tension T1 may be before or after the garment is put on. That is, the tension T1 may be adjusted before the garment is put on to provide the predetermined supporting force as appropriate to the wearer. Alternatively, the garment may be put on before the support belt is attached, and the tension T1 may be adjusted as appropriate to the physical ability and conditions of the wearer on that day or at that time of the day. The tension can thus be adjusted manually in a simple manner. Therefore, once the supporting force is predetermined as appropriate to the physical ability of the wearer under instruction or with the assistance of a trained specialist, the wearer can reproduce the positions of attachment at home and spontaneously work on rehabilitation or effective exercise.

The tension T1 is preferably from 4 N to 55 N. The support belt having a tension T1 of 4 N or more exhibits sufficient resilient force to return to its original shape, thereby appropriately guiding the joint motion toward the desired direction. The support belt having a tension T1 of 55 N or less exhibits moderate pulling force between the two opposite ends, and as a result, the garment is more comfortable to wear, and is capable of reducing the wearer's fatigue from maintaining the posture and body position to wait until joint motion is guided.

The material of the support belt is not particularly limited as long as the support belt contains a material capable of stretching and returning to its original shape, such as a natural rubber, a synthetic plastic and a metal (in the form of a spring) and has appropriate elasticity. However, since the support belt is attached to the garment that people wear, the support belt is preferably made of a material that ensures the safety of the wearer even when they stumble over something. Thus the support belt is preferably made of, for example, an elastic material, such as a rubber and a resin, or a stretchable textile material. The wearer may have some allergy and may be allergic to natural rubber, and therefore the support belt is preferably made of an elastic body such as a synthetic plastic etc., and is preferably made of, for example, the polyester elastomeric resin Hytrel (registered trademark).

The textile material suitable for the support belt may be a stretchable woven or knitted fabric produced by blending elastic fibers (polyurethane fibers, polyester elastomer fibers, etc.) with at least one type of fibers selected from the group consisting of natural fibers, such as silk and wool, regenerated fibers, such as rayon, synthetic fibers, such as acrylic fibers and polyester fibers, and the like; a stretchable woven or knitted fabric produced using elastic fibers alone; or the like. Another textile material suitable for the support belt may be a stretchable polyester woven or knitted fabric containing no elastic fibers but containing, at least in the warp or weft, a multifilament yarn of side-by-side composite fibers consisting of two polyester polymer components divided along the length of the fibers, one component being, for example, a polyester containing polytrimethylene terephthalate (PTT) as a main constituent.

The textile material may be a woven fabric or a knitted fabric, but preferred is a knitted fabric, which is highly stretchable and exhibits sweat-absorbing function, quick-drying function, and other functions due to its knitted structure. The knitting stitches of the knitted fabric may be warp knitting stitches or weft knitting stitches, but preferred is warp knitting stitches, which can produce a high elastic knitted fabric.

The support belt may be made of a single material or a combination of different materials. Different materials may be combined by, for example, stacking and/or connecting different materials having different elongations. By combining different materials in such a manner, the tension can be finely adjusted to the desired level.

The support belt may be in any shape as long as the support belt can be arranged on the body covering to run along a surface of the body of the wearer so that the joint-covering portion of the body covering is positioned between one end of the support belt and the opposite end thereof. However, the circumference of a limb near and around a joint in the human body continuously changes along the length of the limb. Therefore, when the support belt is disposed to cover a body part whose circumference gradually decreases along its length, the support belt is preferably designed to have a tapered shape. The narrower end of the tapered support belt is preferably disposed to cover the body part whose circumference is smaller, and the wider end of the tapered support belt is preferably disposed to cover the body part whose circumference is larger.

The production method of the tapered support belt is not particularly limited, and the tapered support belt can be produced as follows. A single support belt having a uniform width is folded in half such that the support belt partially overlaps itself to make a V-shape to give a tapered support belt whose width becomes gradually smaller from the wider end to the narrower end. The halves of the V-shaped support belt alternately stretch and return to their original shape in response to the pendular motion of a limb around a joint. Due to this function, the V-shaped support belt has several advantages over an I-shape support belt. For example, the tension applied to the belt is moderately dispersed and thus the durability is improved. In addition, since each half of the support belt alternately stretches and returns to its original shape, the narrower end of the belt is prevented from largely shifting in the right or left direction, and the force applied to the support belt is concentrated in the longitudinal direction, thereby stabilizing the desired joint movement. The elongation of the body covering that covers a thick part of a limb inevitably tends to be high. If the width of the support belt is small, the stress by elongation may be concentrated on one location, and further stress may be applied to the body covering, resulting in a tendency of tearing, fraying and breaking of the body covering. According to the present invention, the wider end of the support belt covers a thick part of a limb, and the narrower end of the support belt covers a thin part of the limb, i.e., the tapered support belt is used to cover a limb. In this manner, the support belt prevents the stress from being concentrated on one location, and covers part of the wearer's body in a well-balanced manner so as to be appropriate for the thickness of the body part, thereby allowing the supporting force to act on the body.

The support belts 2a, 2b are desirably disposed obliquely along the surface of the body to extend around the limbs, as shown in Fig. 1 etc. This arrangement allows the use of a relatively long support belt on the limited surface area of the body, leading to accumulation of a large tension force. Here, a limb including a joint of interest and the bones on both sides of the joint is assumed to be in a cylindrical shape, and the edge line extending from said one end 3a to said opposite end 3b of the support belt 2a is denoted as EL as shown in Fig. 1. The angle of the plane containing the starting point of the edge line EL (on said one end 3a) and the center axis of the cylinder with respect to the plane containing the end point of the edge line EL (on said opposite end 3b) and the center axis of the cylinder, i.e., the rotation angle of the support belt with respect to the cylinder, is preferably from 135° to 225°. If the rotation angle of the support belt with respect to the surface of the body, which is assumed to be in a cylindrical shape, is less than 135°, the support belt in a loosened state may create a space between the support belt and the body surface, and a projection such as a stair handrail may be slipped into the space to catch the wearer's body. The support belt having such a small rotation angle is also too short in length to accumulate sufficient tension of the support belt, resulting in difficulty in transferring force to the joint. If the rotation angle of the support belt with respect to the surface of the body, which is assumed to be in a cylindrical shape, is more than 225°, the support belt may excessively rotate around the body surface and may be stuck to the wearer's body, resulting in difficulty in applying sufficient force to the joint.

The above-described configuration of the support belt is preferable in that, by only changing the running direction of the support belt (in the clockwise or counterclockwise direction), the direction of the supporting force for joint movement can be easily changed, which offers a wide choice of training.

The ability of the support belt to elongate is expressed in terms of a spring constant under a load, which shows the elasticity of "a spring". In general, a material with a high spring constant is not easily elongated, whereas a material with a low spring constant is easily elongated by a small force. The support belt preferably has a spring constant of from 0.01 to 0.50 N/mm. The support belt having a spring constant of lower than 0.01 N/mm will have a small spring force for returning to its original shape, and may be poor at guiding the joint motion toward the desired direction. On the other hand, when the support belt has a spring constant of higher than 0.50 N/mm, the pulling force between the opposite ends of the support belt is excessively strong, and as a result, the garment may be difficult to put on, or the wearer may feel exhausted from maintaining the posture and body position to wait until joint motion is guided. Accordingly, the spring constant of the support belt is preferably from 0.01 to 0.50 N/mm. In order to obtain the desired spring constant, a plurality of materials having different spring constants may be stacked on top of each other and/or connected with each other. When two identical materials are stacked on top of each other, the combined spring constant is approximately double that of the single material. When two materials with different spring constants are connected in series, the reciprocal of the combined spring constant is the sum of the reciprocals of the individual spring constants (Hooke's law). Based on these, simulation calculation can be performed to obtain the desired spring constant, which may contribute to shortening the period of time required to determine an appropriate supporting force.

The body covering is preferably made of a stretchable fabric. A poorly stretchable fabric is disadvantageous because the body covering made of a poorly stretchable fabric may create a large space between the body covering and the wearer's body, and the garment may ride up or deform, or the tension of the support belt may not be efficiently transferred to the wearer's body, and as a result, the desired joint motion is difficult to achieve. Therefore the body covering is preferably made of a stretchable fabric to prevent the garment from riding up or deforming, to allow each of the ends of the support belt that are attached to the body covering to serve as the fulcrum of joint movement, and to make the garment tight. The stretchable fabric preferably has an elongation of 30% or more. The stretchable fabric having an elongation of 30% or more is sufficiently conformable to accommodate elongation of the wearer's skin during exercise and thus is highly conformable to accommodate body movement during exercise. A material having anti-slippery function may be used for at least part of the body covering to prevent the garment from riding up. In particular, an anti-slippery fabric, an anti-slippery fabric tape or other products having anti-slippery function may be attached to at least part of the body covering, or a resin may be applied to at least part of the body covering.

The stretchable fabric is preferably a stretchable fabric containing polyurethane elastic fibers. A preferred polyurethane elastic fiber is Lycra (registered trademark) T-127C (trade name), which is commercially available. Polyurethane elastic fibers are advantageous in that they are capable of largely elongating under a small force and quickly returning to their original shape. The body covering is not necessarily required to entirely cover a joint of interest, and part of the body covering may have pores to prevent sweating and to enhance air permeability and comfort of the garment. A meshed fabric is also suitable for the body covering. A part of the body covering that lies below the support belt may have pores.

The joint that is to be supported by the garment of the present invention is not particularly limited, but is preferably the hip joint, which is essential for walking motion. Figs. 1 to 5 show examples of the configuration of the garment of the present invention that supports the hip joint. The garment of the present invention will now be described below with reference to Fig. 1. The garment 20a comprises two support belts 2a, 2b. The body covering 21a comprises a portion (A) 1 for covering the waist of the wearer, a portion (B) 4 for covering the right knee of the wearer, and a portion (C) 5 for covering the left knee of the wearer. The two support belts 2a, 2b are both detachably attached to the body covering. In particular, one end 3a of the support belt 2a is detachably attached to the right side R1 (the outer aspect of the right hip) of the portion A for covering the waist when the garment is worn, and the opposite end 3b of the support belt 2a is detachably attached to the left side L2 (the inner aspect of the right knee) of the portion B for covering the right knee when the garment is worn. One end 3a of the support belt 2b is detachably attached to the left side L1 (the outer aspect of the left hip) of the portion A for covering the waist when the garment is worn, and the opposite end 3b of the support belt 2b is detachably attached to the right side R2 (the inner aspect of the left knee) of the portion C for covering the left knee when the garment is worn. The support belts 2a, 2b are arranged to run along the anterior surface of the thighs.

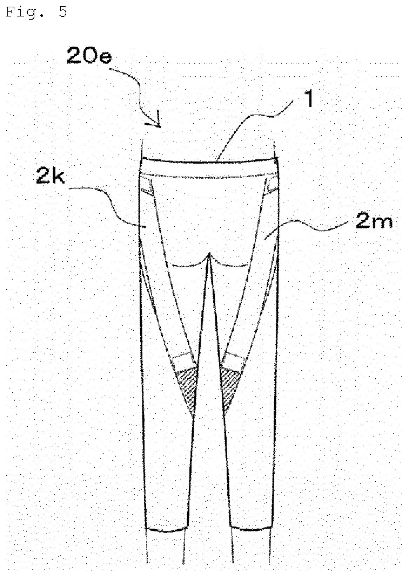

The support belts may be arranged to run along the surface of the body in such a manner that, for example, the support belts 2a, 2b, 2c, 2d, 2e, 2f, 2g, 2h, 2i, 2j run along the anterior surface of the thighs as shown in Figs. 1 to 4, or the support belts 2k, 2m run along the posterior surface of the body as shown in Fig. 5. For individuals with reduced walking ability, the support belts running along the anterior surface of the thighs are preferred. In this arrangement, after the support belts are stretched by hip extension, the tension force accumulated in the support belts is efficiently released by the forward swing of the leg around the hip joint and converted into assisting force. One of the biggest causes of senior stumbles is weakness of the leg's swing force around the hip joint. Appropriate support for the forward swing of the legs around the hip joint will help the stepping of the legs and prevent stumbling and falling. A concern may arise that individuals with reduced walking ability may be required to make considerable effort to accumulate the force in the support belts . However, the wearer does not feel a huge burden of wearing the garment because, in the motion in which one leg is moved forward through hip extension and the other leg is in contact the ground, the hip joint extends due to the inertia of the leg and the shift in the body weight in this natural motion of the body. According to the present invention, the wearer is able to adjust assisting force as appropriate for their walking ability to help their own walking movement produced by their own muscles, thereby effectively preventing loss of muscle strength.

More preferably, one of the support belts may be arranged to run from the outer aspect of the right hip to the inner aspect of the right knee. In an example of the configuration as shown in Fig. 1, the joint of interest is the hip joint. Here, a thigh is assumed to be in a cylindrical shape, and the edge line extending from said one end 3a of the support belt 2a to said opposite end 3b of the support belt 2a is denoted as EL as shown in Fig. 1. The support belt is disposed so that the angle of the plane containing the starting point of the edge line EL (on said one end 3a) and the center axis of the cylinder with respect to the plane containing the end point of the edge line EL (on said opposite end 3b) and the center axis of the cylinder, i.e., the rotation angle of the support belt with respect to the cylinder, is from 175° to 185°. In this arrangement, the length of the support belt is longer than that in cases where, for example, the support belt is arranged to run straight from the outer aspect of the right hip to the outer aspect of the right knee (at an rotation angle of zero degrees), and therefore tension force is more efficiently accumulated. In another arrangement, the support belt may be arranged to obliquely run from an area around the navel of the wearer to the outer aspect of the knee. However, in this arrangement, when the wearer swings the leg around the hip joint to step forward, a space may be relatively easily created between the support belt and the body covering around the crotch. Therefore, the support belt is preferably arranged to run from the outer aspect of the right hip to the inner aspect of the right knee to prevent the creation of a space between the support belt and the body covering during cycles of walking motion. More preferably, the support belt is arranged such that said one end of the support belt is attached to a waist area, around the crest of the pelvis, having a length of 5 cm in the height direction of the wearer and a width of 15 cm in the direction of the circumference of the waist, and said opposite end of the support belt is attached to a knee area, centering around the medial collateral ligament, having a length of 15 cm in the height direction of the wearer and a width of 3 cm in the direction of the circumference of the knee.

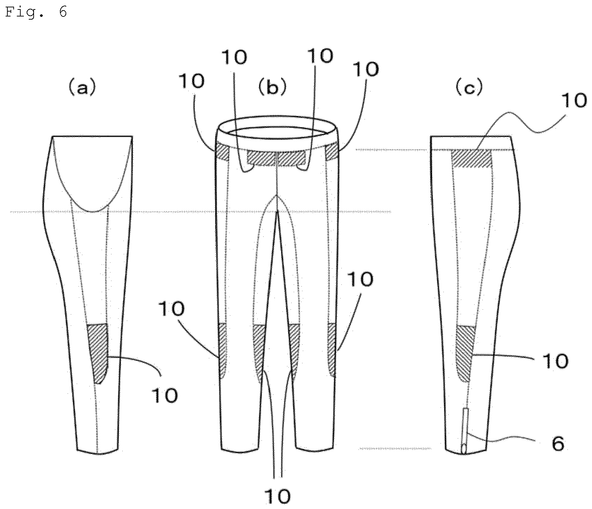

The body covering made of a stretchable fabric is sometimes difficult to put on, and thus the garment preferably has a fastener 6 at the hem as shown in Fig. 6.

Once the physical ability or the activity level of the wearer improves as a result of the walking support, the support belts may be moved to a different area and arranged to run along the back of the body like the support belts 2k, 2m as shown in Fig. 5. In this arrangement, when the wearer swings the leg around the hip joint to step forward (i.e., when the hip joint is flexed), a burden in the opposite direction is applied to the wearer (i.e., assistance for the extension of the hip joint is provided), and in this manner, the wearer can further strengthen the muscles. Needless to say, the present invention is also suitable for enhancing the physical ability of individuals with normal physical ability or a normal activity level, or individuals with relatively high physical ability or a relatively high activity level (athletes, sports players, etc.).

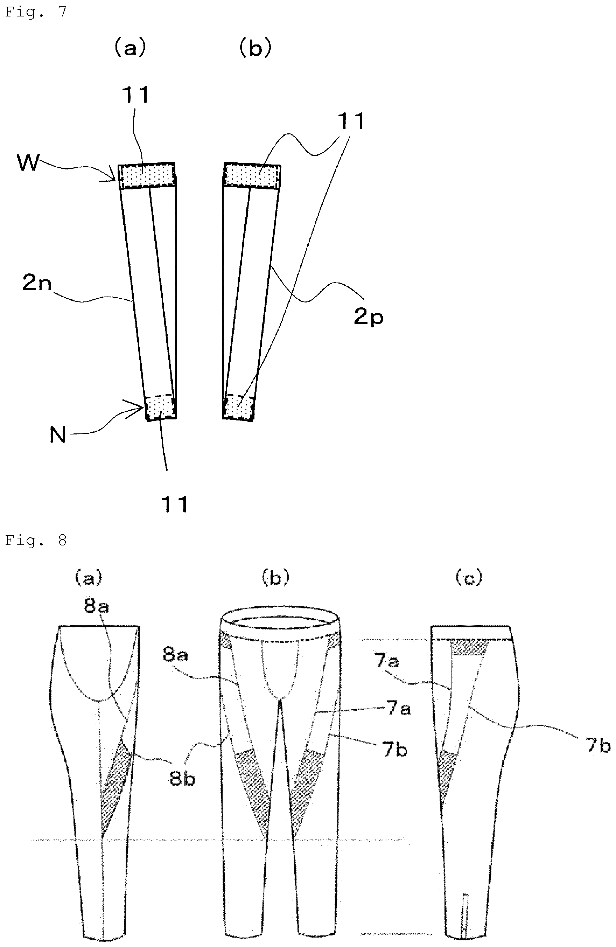

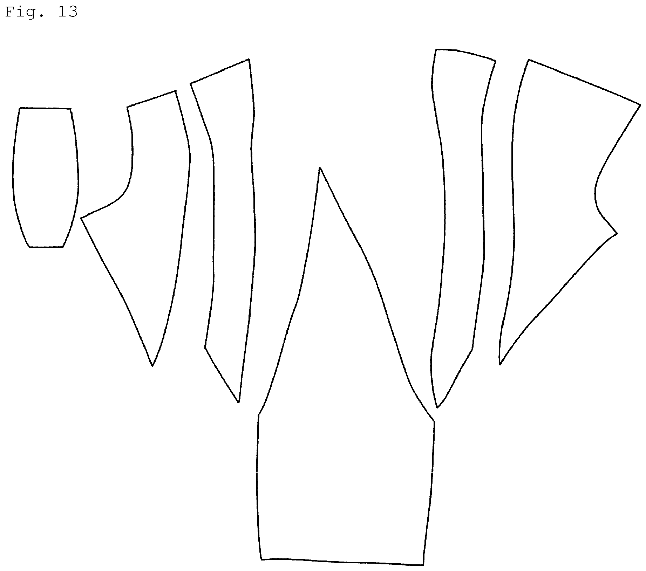

The pattern for making the body covering from a main fabric is not limited, but preferably the locations where the support belts run are indicated on the pattern for the body covering as shown in the cutting pattern of a main fabric shown in Fig. 13 so that the seams of the body covering also serve as guide lines for indicating the locations of the support belts. Alternatively, appropriate length and width of the support belts may be indicated by a series of guide lines 7a, 7b, 8a, 8b provided on the body covering, as shown in an embodiment of the body covering in Fig. 8. The guide lines clarify where to attach the support belts and facilitate adjustment of the tension to the level appropriate for the wearer.

Preferably, the garment of the present invention further contains an adjusting belt for adjusting the waist of the garment of the present invention to fit the wearer's waist. The adjusting belt is overlaid on at least a region around the right and left iliac crests of the wearer and covers the portion (A) 1 for covering the waist shown in Fig. 1. More preferably, the garment of the present invention further contains a hip fastening member that detachably attaches the adjusting belt to at least part of the portion (A) 1 for covering the waist. The adjusting belt is advantageous in that it covers a region around the right and left iliac crests and compresses the wearer's waist, thereby preventing the garment from shifting away from the hip joint area to ensure the tension of the support belts acts on the hip joint. Another advantage exists for the elderly who are not overweight but whose lower abdomen sticks out farther than the pelvis due to falling down of organs into the pelvis as a result of weakening of muscles supporting the organs. In particular, when the body covering is put on (i.e., when the portion A for covering the waist of the wearer (hereinafter also called the waistband) of the body covering is pulled over the wearer's lower abdomen), the body covering provides a sufficient space between the waistband and the wearer's lower abdomen. Then once the body covering is put on and the waistband is placed on the waist of the wearer, the waistband is fastened against the wearer's waist, thereby sufficiently reducing the space between the waistband and the wearer's waist. The adjusting belt is preferably made of a stretchable fabric, and the stretchable fabric may be the same as those described above. The hip fastening member may be composed of, for example, a fastening member disposed on the portion (A) 1 for covering the waist and another fastening member disposed on the adjusting belt, and the fastening member on the adjusting belt is secured to the fastening member on the portion (A) 1 for covering the waist. The fastening members may be hook and loop fasteners. When the garment is worn by the elderly whose abdomen sticks out farther than the pelvis due to falling down of organs into the pelvis as a result of weakening of muscles supporting the organs, the distance between the crotch to the waist of the body covering is preferably long enough such that the body covering covers the whole lower abdomen, thereby preventing the body covering from slipping down from the lower abdomen.

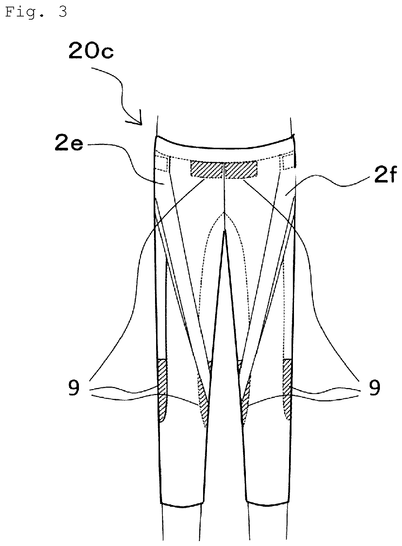

Fig. 3 shows a schematic front view of the garment according to another embodiment of the present invention when it is worn. The garment 20c has a body covering to which at least four support belts can be attached. In the figure, two support belts 2e, 2f are attached to the body covering. The support belt 2e is attached to the body covering in such a manner that it runs along the wearer's body from the outer aspect of the right hip to the inner aspect of the right knee. The support belt 2f is attached to the body covering in such a manner that it runs along the wearer's body from the outer aspect of the left hip to the inner aspect of the left knee. In this embodiment, the hook or loop portions of a hook and loop fastener are provided to the support belts to be paired with the corresponding hook or loop portions on the body covering serving as fastening members 9 so that the support belts are attached to the body covering via the paired hook and loop portions. Of the fastening members 9 in this embodiment, which are the hook or loop portions to be paired with those on the support belts, the fastening members 9 that are disposed on the inner knee-covering portions of the body covering (hereinafter also called inner fastening members) and the fastening members 9 that are disposed on the outer knee-covering portions of the body covering (hereinafter also called outer fastening members) have a relatively elongated shape in the height direction of the wearer. In this arrangement, the ends of the support belts to be disposed on the inner aspect of the knees can be attached to selected positions within the inner fastening members 9, and thereby the wearer can adjust the positions of the ends in the height direction of the wearer. In this manner, the tension T1 of the support belts can be adjusted to the desired level.

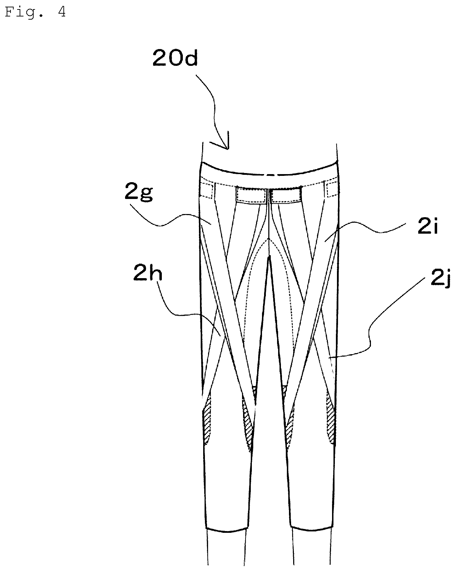

Fig. 4 shows a schematic front view of the garment according to another embodiment of the present invention when it is worn. The body covering of the garment according to this embodiment is the same as that in the embodiment of the garment shown in Fig. 3. The garment of this embodiment differs from that shown in Fig. 3 in that it has two more additional support belts, i.e., a total of four support belts 2g, 2h, 2i, 2j are attached to the body covering.

Fig. 5 shows a schematic rear view of the garment according to another embodiment of the present invention when it is worn. In this embodiment of the garment 20e, two support belts 2k, 2m are attached to the back of the body covering of the garment 20e. The support belt 2m is attached to the body covering such that it runs along the wearer's body from the outer aspect of the right hip to the inner aspect of the right knee. The support belt 2k is attached to the body covering such that it runs along the wearer's body from the outer aspect of the left hip to the inner aspect of the left knee.

Fig. 6 (a) shows a schematic left side view of the body covering used in the embodiment of the garment of the present invention as shown in Figs. 3 and 4. Fig. 6 (b) shows a schematic front view of the body covering used in the embodiment of the garment of the present invention as shown in Figs. 3 and 4. Fig. 6 (c) shows a schematic right side view of the body covering used in the embodiment of the garment of the present invention as shown in Figs. 3 and 4. The body covering has two fastening members 10 on the abdominal area, has one fastening member 10 on each of the outer aspect of the right hip and the outer aspect of the left hip, has one fastening member 10 on each of the outer and inner aspects of the right knee, and has one fastening member 10 on each of the outer and inner aspects of the left knee.

Fig. 7 (a) and (b) shows a schematic front view of the support belts used in the embodiment of the garment of the present invention as shown in Figs. 1 to 5. The support belts 2n, 2p are tapered support belts whose width becomes gradually smaller from the wider end W to the narrower end N. The tapered support belts are each produced by folding a single support belt having a uniform width in half such that the support belt partially overlaps itself to make a V-shape. The tapered support belts 2n, 2p each have fastening members 11 on both ends, which are hook and loop fasteners to be paired with the corresponding fastening members.

Fig. 8 (a) is a schematic left side view of the body covering used in the embodiment of the garment of the present invention as shown in Figs. 1 and 2. Fig. 8 (b) is a schematic front view of the body covering used in the embodiment of the garment of the present invention as shown in Figs. 1 and 2. Fig. 8 (c) is a schematic right side view of the body covering used in the embodiment of the garment of the present invention as shown in Figs. 1 and 2. The body covering has guide lines 7a, 7b, 8a, 8b for indicating appropriate locations for attachment of the support belts to the body covering.

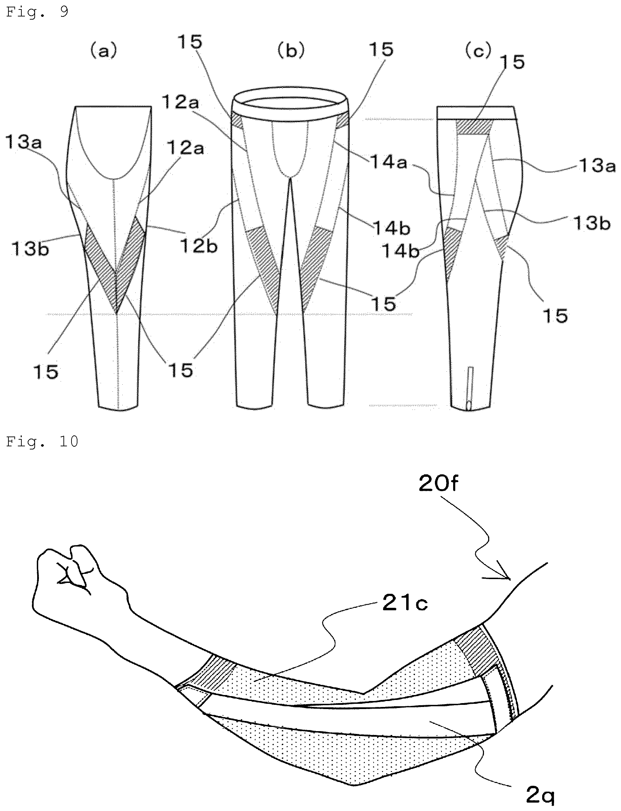

Fig. 9 (a) is a schematic left side view of the body covering used in the embodiment of the garment of the present invention as shown in Fig. 5. Fig. 9 (b) is a schematic front view of the body covering used in the embodiment of the garment of the present invention as shown in Fig. 5. Fig. 9 (c) is a schematic right side view of the body covering used in the embodiment of the garment of the present invention as shown in Fig. 5. Guide lines for indicating appropriate locations for attachment of the support belts are provided not only on the front but also on the back of the body covering. The numerals 12a, 12b, 13a, 13b, 14a, and 14b indicate the guide lines, and the numeral 15 indicates the fastening members.

Fig. 10 shows a schematic right side view of the garment according to another embodiment of the present invention when it is worn. The joint that is supported by the garment 20f of this embodiment is an elbow joint. In other words, the body covering 21c of the garment 20f covers the wearer's elbow joint and the area around the joint. The support belt 2q of the garment 20f is arranged on the body covering to run along a surface of the wearer's body so that the elbow-covering portion of the body covering is positioned between one end of the support belt 2q and the opposite end thereof when the garment 20f is worn.

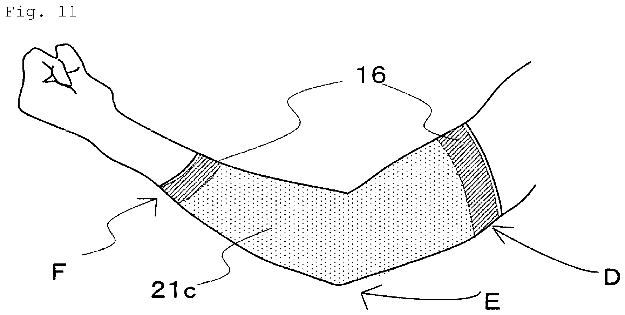

Fig. 11 shows a schematic right side view of the body covering 21c used in the embodiment of the garment 20f of the present invention as shown in Fig. 10. The body covering 21c has fastening members 16 on both ends, which are hook and loop fasteners to be paired with the corresponding fastening members.

The body covering 21c for covering an elbow joint is a tubular body comprising a portion D for covering an upper arm of the wearer, a portion E for covering an elbow of the wearer, and a portion F for covering a wrist of the wearer. Preferably, said one end of the support belt 2q is detachably attached to the portion D when the garment 20f is worn, and said opposite end of the support belt 2q is detachably attached to the portion F when the garment 20f is worn. In this arrangement, the wearer can freely adjust the supporting force and the angle of the support belt to the desired level without need of excessively rotating the elbow or twisting the arm. In this manner, joint movement as appropriate for the physical strength and function of the wearer is able to be achieved.

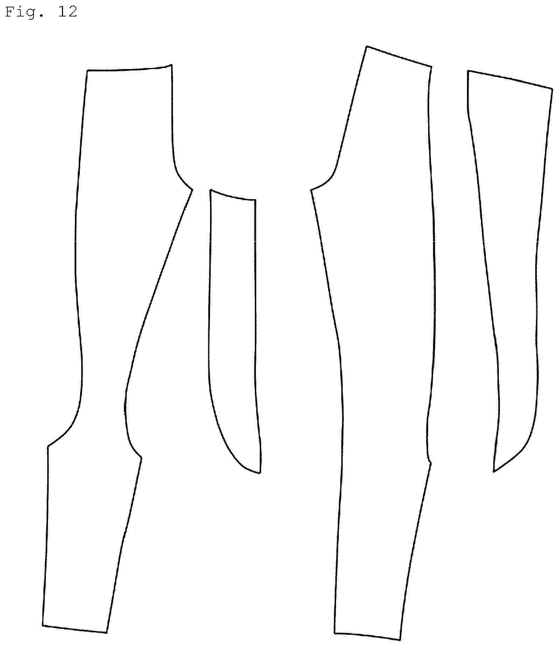

Fig. 12 shows a schematic developed view of the pieces of the body covering used in the embodiment of the garment of the present invention as shown in Fig. 6 (a), (b) and (c). These pieces are sewn into the body covering used in the embodiment of the garment of the present invention as shown in Fig. 6 (a), (b) and (c).

Fig. 13 shows a schematic developed view of the pieces of the body covering used in the embodiment of the garment of the present invention as shown in Fig. 8 (a), (b) and (c). These pieces are sewn into the body covering used in the embodiment of the garment of the present invention as shown in Fig. 8 (a), (b) and (c).

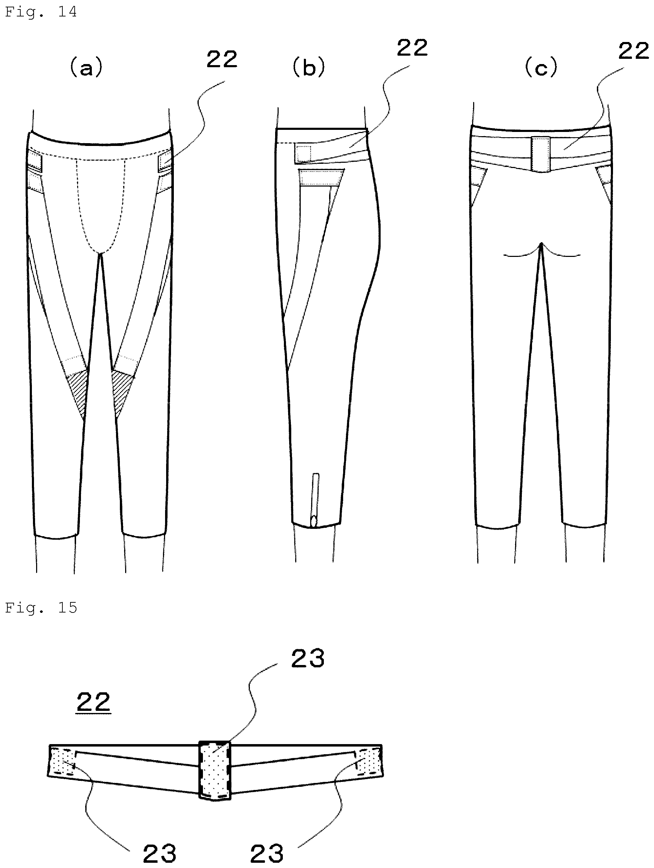

Fig. 14 (a) shows a schematic front view of the garment according to another embodiment of the present invention when it is worn. Fig. 14 (b) shows a schematic right side view of the garment according to the embodiment of the present invention when it is worn. Fig. 14 (c) shows a schematic rear view of the garment according to the embodiment of the present invention when it is worn. The garment has an adjusting belt 22 for adjusting the circumference of the waist-covering portion A of the body covering. One end of each of the support belts is overlaid on and attached to a right and left iliac crest-covering portion of the body covering for covering a region around the iliac crests of the wearer when the garment is worn, so that a portion of each support belt between its ends covers the wearer's hip. Preferably, one end of each of the support belts is attached to the right and left iliac crest-covering portion of the body covering for covering a region around the iliac crests of the wearer, desirably attached to the right and left top iliac crest-covering portion of the body covering, when the garment is worn, so that said end of each of the support belts is caught on the top of the pelvis of the wearer when the adjusting belt is fastened. Due to this configuration of the garment as shown in Fig. 14 (a), (b) and (c), during walking or other exercise of the wearer, the waistband being pulled down by the tension of the support belts disposed on the thighs is prevented from gradually slipping down, and in this manner, the body covering and the support belts are maintained at appropriate positions.

Fig. 15 shows a schematic front view of an adjusting belt according to an embodiment of the present invention, used in the garment of the present invention as shown in Fig. 14. The width of the adjusting belt 22 becomes gradually larger from the ends to the center. The adjusting belt 22 has the fastening members 23 on the both ends and the center, which are hook and loop fasteners to be paired with the corresponding fastening members.

EXAMPLES

The present invention will be described in detail below with reference to Examples.

Elongation (%)

Elongation (%) was determined in accordance with JIS L 1096 (2010) "Testing methods for woven and knitted fabrics". A specimen was clamped at a distance of 20 cm and elongated up to a tensile load of 14.7 N at a tensile speed of 20 cm/min using a constant-rate-of-extension tester as specified in the JIS standard to determine elongation (%) in the wale and course directions.

Elongation recovery (%)

Elongation recovery (%) in the wale and course directions was determined in accordance with the B-1 method specified in JIS L 1096 (2010) "Testing methods for woven and knitted fabrics". A specimen was clamped on one end and hung from the clamp. The clamped position and the position 20 cm below the lower end of the clamp were marked on the specimen. A load of 14.7 N was applied to the other end of the specimen, and the specimen was held stationary for one hour. Then the length between the marked positions was measured. The load was removed and the specimen was left to stand for 30 seconds. The length between the marked positions was measured again to determine elongation recovery (%).

Spring constant

A support belt was elongated in the longitudinal direction under a load of 4.9 N at a constant speed (1000 mm/min) with a TENSILON tensile tester. The gauge length was the length of the non-attached portion of the support belt. A stress-strain curve at 30% more elongation relative to the initial length was plotted, and the spring constant (N/mm) was calculated from the slope of the stress-strain curve.

Tensions T1 and T2

The garment of the present invention was put on, and the edges of the opposite ends of the support belt that were attached to the body covering were marked on the garment without loosening the support belt and the body covering. The distance between the marks was measured along the body surface to determine the length of the non-attached portion of the support belt in the longitudinal direction. This length was denoted as length X. Then, the garment was taken off, and the length of the non-attached portion of the support belt and the length of the portion of the body covering that was below the non-attached portion of the support belt (i.e., the distance between the marks) were measured in the absence of tension. When the garment is not worn, the length of the portion of the body covering that is to be below the non-attached portion of the support belt is longer than that of the non-attached portion of the support belt, and the portion of the body covering is more loosened than the non-attached portion of the support belt. The length of the non-attached portion of the support belt measured in the longitudinal direction when the garment is not worn was denoted as length Y. The length of the portion of the body covering that was below the non-attached portion of the support belt measured in the longitudinal direction when the garment is not worn was denoted as length Z.

The tension T1 was measured using a TENSILON universal material testing instrument RTG-1210 (A&D Company) with a load cell of 250 N. The support belt was clamped between two aluminum plates (10 cm in width $\times$ 5 cm in length $\times$ 0.5 mm in thickness) at both ends at a clamping distance of length Y. The support belt was elongated from length Y to 125% of length X at 1000 mm/min. A tension (N)-strain curve was plotted, and the tension (N) at the strain X was determined from the curve. The experiment was repeated three times and the arithmetic mean value was taken as the tension T1.

The tension T2 was measured on a specimen prepared by cutting the portion of the body covering that was below the support belt to a size equal to that of the support belt. The specimen was clamped between two aluminum plates (10 cm in width $\times$ 5 cm in length $\times$ 0.5 mm in thickness) at both ends at a clamping distance of length Z. The support belt was elongated from length Z to 125% of length X at 1000 mm/min. A tension (N)-strain curve was plotted, and the tension (N) at the strain X was determined from the curve. The experiment was repeated three times and the arithmetic mean value was taken as the tension T2. In the above manner, the tensions T1 and T2 when the support belt is attached to the body covering were determined in simulated conditions.

Motion analysis

To examine the effect of the support belt, an analysis was performed on five subjects (A: 25-year-old man, B: 44-year-old man, C: 78-year-old man, D: 74-year-old woman, and E: 40-year-old woman) . The subjects were ordered to raise one foot and put it down in front of the other at a sign from the conductor, then raise the other foot and put it next to the other, and stand straight. The movement of the knee joint of the leg that was moved first was measured using a depth sensor (trade name: "Kinect", Microsoft Corporation). Joint motion analysis was performed on the information obtained by the measurement. The analysis was focused on the moving distance of the knee joint. The moving distance of the knee joint was determined in experiments using four experimental movements as described below, and the results were compared. A greater moving distance of the knee joint indicates more effective support for forward leg swing around the hip joint.

In examination of the effect of the support belt in the Examples below, the support belt was attached only to the right leg of the subjects. The subjects were ordered to take a step forward with the same level of force as usual, as much as they could. The following four experimental movements (a) to (d) were performed in the following order, and the measurement was carried out seven times for each movement:

(a) the subject raises the right foot and puts it down in front of the other without wearing the support belt;
(b) the subject raises the left foot and puts it down in front of the other without wearing the support belt;
(c) the subject raises the right foot and puts it down in front of the other with wearing the support belt only on the right leg; and
(d) the subject raises the left foot and puts it down in front of the other with wearing the support belt only on the right leg.

The type of the support belt for the subjects were selected as appropriate for the physical ability of each subject after trial and error.

The spring constant, the tensions T1 and T2, and the T1/T2 ratio of the support belt used for the five subjects are shown in Table 1 below.

The knee movement was measured with the above-mentioned depth sensor (frame rate: 30 Hz) to obtain the time series data sets of the three-dimensional position of the knee joint of the leg that was first moved by the subjects, as represented by formula (1) below. In the formulas below, n is the frame number. In the assessment of the moving distance of the knee joint, the position of the knee joint at the start of the measurement (the first frame of the data) was defined as the starting point. For each of the data sets obtained from the experimental movements (a) to (d) by measuring seven times, the maximum value $d_{knee}$ of the relative distance from the starting point to the knee joint position during the movement was extracted by the formula (2) below. The arithmetic mean value of the seven maximum values $d_{knee}$ of the relative distance obtained from the seven times of measurement was taken as the moving distance of the knee joint.

Formula (1)

$$P_{knee}(n) = [x_{knee}(n), y_{knee}(n), z_{knee}(n)]$$

Formula (2)

$$d_{knee} = \max[[x_{knee}(n)^2 + y_{knee}(n)^2 + z_{knee}(n)^2]^{1/2}]$$

Example 1

A nylon yarn and a polyurethane elastic fiber (Lycra (registered trademark), trade name "T-127C") yarn were knitted in a tricot pattern and then dyed to give a stretchable fabric containing 70% of nylon and 30% of polyurethane (mass per unit area: 250 g/m$^2$). The elongation was 140% in the wale direction and 100% in the course direction. The elongation recovery was 92% in the wale direction and 86% in the course direction. The pieces of a garment as shown in Fig. 12 were cut out from the fabric and were sewn together with a flat seam sewing machine to give an undergarment as shown in Fig. 6. Stretchable loop (female) fasteners were attached to the areas indicated by oblique lines in Fig. 6.

Then, a nylon yarn and a polyurethane elastic fiber (Lycra (registered trademark), trade name "T-127C") yarn were knitted and then dyed to give a power net fabric. From the power net fabric, strips of 76 cm in length and 5 cm in width with the longer dimensions parallel to the longitudinal direction of the fabric were cut out. Each strip was folded in half such that the strip partially overlaps itself to make a V-shape. To one end, a loop (male) fastener of 5 cm in length and 5 cm in width (YKK Corporation) was attached by sewing, and to the opposite end, a loop (male) fastener of 5 cm in length and 8 cm in width (YKK Corporation) was attached by sewing. In this manner, tapered support belts whose width becomes gradually smaller from the wider end to the narrower end as shown in Fig. 7 were produced. The length of the non-attached portion of each of the tapered support belts excluding the length of the loop fasteners was 28.5 cm. (The total length of each of the tapered support belts including the length of the loop fasteners at both ends was 38.5 cm.)

Subject A was ordered to put on the undergarment. The tapered support belts were attached to the undergarment as shown in Fig. 3 while moderate tension was applied to the support belts so that the non-attached portions of the support belts were stretched from the initial length of 28.5 cm to 48.8 cm. Motion analysis was performed on the subject wearing the undergarment to determine the moving distance (cm) of the knee joints. The results are shown in Table 2 below.

Comparative Example 1

Subject A was ordered to put on the same undergarment as that in Example 1. Motion analysis was performed without attaching the support belts to determine the moving distance (cm) of the knee joints. The results are shown in Table 2.
As shown in Table 2, the moving distance of the knee joints in Example 1 was longer than that in Comparative Example 1.

Example 2

Subject B was ordered to put on the same undergarment as that in Example 1. The tapered support belts were attached to the undergarment as shown in Fig. 3 while moderate tension was applied to the support belts so that the non-attached portions of the support belts were stretched from the initial length of 28.5 cm to 48.7 cm. Motion analysis was performed on the subject wearing the undergarment to determine the moving distance (cm) of the knee joints. The results are shown in Table 2 below.

Comparative Example 2

Subject B was ordered to put on the same undergarment as that in Example 1. Motion analysis was performed without attaching the support belts to determine the moving distance (cm) of the knee joints. The results are shown in Table 2.
As shown in Table 2, the moving distance of the knee joints in Example 2 was longer than that in Comparative Example 2.

Example 3

A nylon yarn and a polyurethane elastic fiber (Lycra (registered trademark), trade name "T-906C") yarn were knitted in a tricot pattern and then dyed to give a stretchable fabric containing 70% of nylon and 30% of polyurethane (mass per unit area: 200 g/m$^2$). The elongation was 120% in the wale direction and 95% in the course direction. The elongation recovery was 93% in the wale direction and 92% in the course direction. The pieces of a garment as shown in Fig. 13 were cut out from the fabric and were sewn together with a flat seam sewing machine to give an undergarment as shown in Fig. 8. Stretchable loop (female) fasteners were attached to the areas indicated by oblique lines in Fig. 8.
A rubber-based tape of 5 cm in width was cut into pieces of 76 cm in length. Each tape was folded in half such that the tape partially overlaps itself to make a V-shape. To one end, a loop (male) fastener of 5 cm in length and 5 cm in width (YKK Corporation) was attached by sewing, and to the opposite end, a loop (male) fastener of 5 cm in length and 8 cm in width (YKK Corporation) was attached by sewing. In this manner, tapered support belts whose width becomes gradually smaller from the wider end to the narrower end as shown in Fig. 7 were produced. The length of the non-attached portion of each of the tapered support belts excluding the length of the loop fasteners was 28.5 cm.
Subject C was ordered to put on the undergarment. The tapered support belts were attached to the undergarment as shown in Fig. 1 while moderate tension was applied to the support belts so that the non-attached portions of the support belts were stretched from the initial length of 28.5 cm to 48.5 cm. Motion analysis was performed on the subject wearing the undergarment to determine the moving distance (cm) of the knee joints. The results are shown in Table 2 below.

Comparative Example 3

Subject C was ordered to put on the same undergarment as that in Example 3. Motion analysis was performed without attaching the support belts to determine the moving distance (cm) of the knee joints. The results are shown in Table 2.
As shown in Table 2, the moving distance of the knee joints in Example 3 was longer than that in Comparative Example 3.

Example 4

Subject D was ordered to put on the same undergarment as that in Example 3. The tapered support belts were attached to the undergarment as shown in Fig. 4 while moderate tension was applied to the support belts so that the non-attached portions of the support belts were stretched from the initial length of 28.5 cm to 48.9 cm. Motion analysis was performed on the subject wearing the undergarment to determine the moving distance (cm) of the knee joints. The results are shown in Table 2 below.

Comparative Example 4

Subject D was ordered to put on the same undergarment as that in Example 3. Motion analysis was performed without attaching the support belts to determine the moving distance (cm) of the knee joints. The results are shown in Table 2.
As shown in Table 2, the moving distance of the knee joints in Example 4 was longer than that in Comparative Example 4.

Example 5

A nylon yarn and a polyurethane elastic fiber (Lycra (registered trademark), trade name "T-906C") yarn were knitted in a tricot pattern and then dyed to give a stretchable fabric containing 70% of nylon and 30% of polyurethane (mass per unit area: 250 g/m$^2$). The elongation was 95% in the wale direction and 120% in the course direction. The elongation recovery was 93% in the wale direction and 92% in the course direction. The pieces of a garment as shown in Fig. 13 were cut out from the fabric and were sewn together with a flat seam sewing machine to give an undergarment as shown in Fig. 8. Stretchable loop (female) fasteners were attached to the areas indicated by oblique lines in Fig. 8.
Then, a nylon yarn and a polyurethane elastic fiber (Lycra (registered trademark), trade name "T-127C") yarn were knitted and then dyed to give a power net fabric. From the power net fabric, strips of 76 cm in length and 5 cm in width with the longer dimensions parallel to the longitudinal direction of the fabric were cut out. Each strip was folded in half such that the strip partially overlaps itself to make a V-shape. To one end, a loop (male) fastener of 5 cm in length and 5 cm in width (YKK Corporation) was attached by sewing, and to the opposite end, a loop (male) fastener of 5 cm in length and 8 cm in width (YKK Corporation) was attached by sewing. In this manner, tapered support belts whose width becomes gradually smaller from the wider end to the narrower end as shown in Fig. 7 were produced. The length of the non-attached portion of each of the tapered support belts excluding the length of the loop fasteners was 28.5 cm. (The total length of each of the tapered support belts including the length of the loop fasteners at both ends was 38.5 cm.)
Subject E was ordered to put on the undergarment. The tapered support belts were attached to the undergarment as shown in Fig. 3 while moderate tension was applied to the support belts so that the non-attached portions of the support belts were stretched from the initial length of 28.5 cm to 47.5 cm. Motion analysis was performed on the subject wearing the undergarment to determine the moving distance (cm) of the knee joints. The results are shown in Table 2 below.

Comparative Example 5

Subject E was ordered to put on the same undergarment as that in Example 5. Motion analysis was performed without attaching the support belts to determine the moving distance (cm) of the knee joints. The results are shown in Table 2.
As shown in Table 2, the moving distance of the knee joints in Example 5 was longer than that in Comparative Example 5.

Example 6

The same undergarment as that in Example 5 was used. The same power net fabric as that in Example 5 was used for making support belts. From the power net fabric, strips of 40 cm in length and 5 cm in width with the longer dimensions parallel to the longitudinal direction of the fabric were cut out. Each strip was folded in half such that the strip partially overlaps itself to make a V-shape. To one end, a loop (male) fastener of 2.5 cm in length and 5 cm in width (YKK Corporation) was attached by sewing, and to the opposite end, a loop (male) fastener of 2.5 cm in length and 8 cm in width (YKK Corporation) was attached by sewing. In this manner, tapered support belts whose width becomes gradually smaller from the wider end to the narrower end as shown in Fig. 7 were produced. The length of the non-attached portion of each of the tapered support belts excluding the length of the loop fasteners was 15.0 cm. (The total length of each of the tapered support belts including the length of the loop fasteners at both ends was 20.0 cm.)
Subject E was ordered to put on the undergarment. The tapered support belts were attached to the undergarment as shown in Fig. 2 while moderate tension was applied to the support belts so that the non-attached portions of the support belts were stretched from the initial length of 15.0 cm to 20.5 cm. Motion analysis was performed on the subject wearing the undergarment to determine the moving distance (cm) of the knee joints. The results are shown in Table 2 below.

Comparative Example 6

Subject E was ordered to put on the same undergarment as that in Example 5. Motion analysis was performed without attaching the support belts to determine the moving distance (cm) of the knee joints. The results are shown in Table 2.

As shown in Table 2, the moving distance of the knee joints in Example 6 was longer than that in Comparative Example 6.

Table 1

|  |  | Spring constant (N/mm) | Tension T1 (N) | Tension T2 (N) | T1/T2 |
|---|---|---|---|---|---|
| Subject A | Example 1 | 0.032 | 13.2 | 1.2 | 11 |
| Subject B | Example 2 | 0.032 | 12.6 | 1.6 | 7.8 |
| Subject C | Example 3 | 0.018 | 40.7 | 0.75 | 54 |
| Subject D | Example 4 | 0.018 | 42.4 | 0.55 | 77 |
| Subject E | Example 5 | 0.018 | 11.9 | 0.69 | 17.2 |
| Subject E | Example 6 | 0.018 | 3.1 | 0.34 | 9.1 |

Table 2

|  |  | Moving distance of knee joints without wearing support belts (cm) | | Moving distance of knee joints with wearing support belt only on right leg (cm) | | |
|---|---|---|---|---|---|---|
|  |  | Left leg | Right leg |  | Left leg | Right leg |
| Subject A | Comparative Example 1 | 63 | 63 | Example 1 | 65 | 71 |
| Subject B | Comparative Example 2 | 60 | 59 | Example 2 | 60 | 64 |
| Subject C | Comparative Example 3 | 41 | 39 | Example 3 | 39 | 42 |
| Subject D | Comparative Example 4 | 59 | 60 | Example 4 | 61 | 62 |
| Subject E | Comparative Example 5 | 51 | 50 | Example 5 | 54 | 54 |
| Subject E | Comparative Example 6 | 51 | 50 | Example 6 | 53 | 53 |

REFERENCE SIGNS LIST

1 Portion (A) for covering the waist of a wearer
2a, 2b, 2c, 2d, 2e, 2f, 2g, 2h, 2i, 2j, 2k, 2m, 2n, 2p, 2q Support belt
3a One end of support belt
3b Opposite end of support belt
4 Portion (B) for covering the right knee of a wearer
5 Portion (C) for covering the left knee of a wearer
6 Fastener
7a, 7b Guide line
8a, 8b Guide line
9 Fastening member
10 Fastening member

11 Fastening member
12a, 12b Guide line
13a, 13b Guide line
14a, 14b Guide line
15 Fastening member
16 Fastening member
20a, 20b, 20c, 20d, 20e Garment
21a, 21b, 21c Body covering
22 Adjusting belt
23 Fastening member to be paired with the corresponding fastening member
D Portion for covering an upper arm of a wearer
E Portion for covering an elbow of a wearer
F Portion for covering a wrist of a wearer
EL Edge line of support belt
R1 Right side of portion (A) for covering the waist
L1 Left side of portion (A) for covering the waist
R2 Right side of portion (C) for covering the left knee
L2 Left side of portion (B) for covering the right knee

**Claims**

1.  A garment comprising a body covering and a support belt arranged on the body covering, the body covering comprising a portion for covering a joint of a wearer,
    wherein the support belt is arranged on the body covering to run along a surface of the body of the wearer so that the joint-covering portion of the body covering is positioned between one end of the support belt and the opposite end thereof,
    wherein the support belt is attached to the body covering so that the support belt and the body covering are separately and individually stretchable, and
    wherein the support belt has a tension T1 in a direction S connecting said one end and said opposite end of the support belt, and a portion of the body covering that lies below the support belt has a tension T2 in a direction parallel to the direction S, and the tension T1 is larger than the tension T2 when the garment is worn.

2.  The garment according to claim 1, which has a ratio of the tension T1 to the tension T2 (T1/T2) ranging from 3 to 100.

3.  The garment according to claim 1 or 2, which further has a means for adjusting the tension T1.

4.  The garment according to any one of claims 1 to 3, which comprises two support belts A1, A2,
    wherein the body covering comprises a portion A for covering the waist of the wearer, a portion B for covering the right knee of the wearer and a portion C for covering the left knee of the wearer,
    wherein one end of the support belt A1 is detachably attached to the portion A, and the opposite end of the support belt A1 is detachably attached to the portion B, and
    wherein one end of the support belt A2 is detachably attached to the portion A, and the opposite end of the support belt A2 is detachably attached to the portion C.

5.  The garment according to any one of claims 1 to 4, which further comprises an adjusting belt,
    wherein the body covering comprises a portion A for covering the waist of the wearer and a portion for covering a region around the right and left iliac crests of the wearer when the garment is worn,
    wherein the adjusting belt is overlaid on the iliac crest-covering portion of the body covering, and
    wherein the garment has a hip fastening member that detachably attaches the adjusting belt to at least part of the portion A.

6.  The garment according to any one of claims 1 to 3, wherein the body covering is a tubular body comprising a portion D for covering an upper arm of the wearer, a portion E for covering an elbow of the wearer, and a portion F for covering a wrist of the wearer, and
    wherein one end of the support belt is detachably attached to the portion D, and the opposite end of the support belt is detachably attached to the portion F.

Fig. 1

Fig. 2

Fig. 3

20c

2e

2f

9        9

Fig. 4

Fig. 5

Fig. 6

(a)

(b)

(c)

10 10 10 10

10

10

10

10

10

10

10

10

6

Fig. 7

(a)     (b)

11

W

2n

N

11

11

2p

Fig. 8

(a)     (b)     (c)

8a

8a

8b

7a

7a

7b

7b

Fig. 9

(a)    (b)    (c)

13a    15    12a    15    15

12a

13b    12b    14a

14b    13a

13b

14b    14b

15    15    15    15

Fig. 10

20f

21c

2q

Fig. 11

16

F

21c

D

E

Fig. 12

Fig. 13

Fig. 14

(a) 22     (b) 22     (c) 22

Fig. 15

22

23

23     23

## INTERNATIONAL SEARCH REPORT

| International application No. |
|---|
| PCT/JP2017/012154 |

**A. CLASSIFICATION OF SUBJECT MATTER**
*A41D13/05*(2006.01)i, *A41D13/08*(2006.01)i, *A61H3/00*(2006.01)i

According to International Patent Classification (IPC) or to both national classification and IPC

**B. FIELDS SEARCHED**

Minimum documentation searched (classification system followed by classification symbols)
A41D13/00-13/12, A61H3/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2015-172255 A (Mizuno Inc.), 01 October 2015 (01.10.2015), paragraphs [0035] to [0041]; fig. 9 to 10 & JP 5947328 B2 | 1-3,5 |
| X | WO 2008/122873 A1 (DY & VY S.R.L.), 16 October 2008 (16.10.2008), description, page 5, line 26 to page 6, line 22; fig. 1 to 2 & IT MI20070706 A1 | 1,3-4 |
| X | JP 2001-32108 A (Daiya Industry Co., Ltd.), 06 February 2001 (06.02.2001), paragraphs [0007] to [0010]; fig. 1 to 7 & JP 3587742 B2 | 1,6 |

☐ Further documents are listed in the continuation of Box C.     ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 05 June 2017 (05.06.17) | 13 June 2017 (13.06.17) |

| Name and mailing address of the ISA/ | Authorized officer |
|---|---|
| Japan Patent Office 3-4-3,Kasumigaseki,Chiyoda-ku, Tokyo 100-8915,Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H10130915 A
- JP H10280209 A
- JP 2006219778 A
- JP 2013227717 A
- JP 2009095645 A
- JP 2014018536 A